# EUROPEAN PATENT APPLICATION

(11) **EP 3 767 290 A1**
(43) Date of publication of application: **20.01.2021**
(21) Application number: 19187336.3
(22) Date of filing: 19.07.2019
(51) Int. Cl.: G01N 33/00

(54) **METHOD AND CALIBRATION UNIT FOR CALIBRATING A CONSTANT VOLUME SAMPLING SYSTEM FOR ANALYZING EXHAUST EMISSIONS**

(71) Applicant: L'Air Liquide Société Anonyme pour l'Etude et l'Exploitation des Procédés Georges Claude, 75007 Paris (FR); Air Liquide UK Limited, Birmingham B46 1JY (GB)
(72) Inventor: Morrey, William, Birmingham, B46 1JY (GB); Vasarhelyi, Martin, 2630 TAASTRUP (DK); Millward, David, Birmingham, B46 1JY (GB); Hopper, David, Birmingham, B46 1JY (GB); Carré, Martine, 78350 LES LOGES EN JOSAS (FR)
(74) Representative: Keenway Patentanwälte Neumann Heine Taruttis

(57) **Abstract**

The calibration unit 1 for calibrating a constant volume sampling (CVS) system comprises a heating jacket 2 by which the carbon dioxide container 4 and the carbon dioxide therein can be heated before the provision to the CVS system starts avoiding blocking the system due to solid carbon dioxide being generated due to the temperature drop originating in the expansion of the carbon dioxide during extraction from the compressed carbon dioxide container 4.

The use of carbon dioxide as a calibration gas for the CVS process has significant advantages compared to the use of propane for the calibration process. On the one hand the legal requirements for carbon dioxide are more harmonized than for propane, in particular in the European Union and the United States of America are identical. On the other hand the use of carbon dioxide increases the measurement accuracy in particular for carbon dioxide which is nowadays one of the most important measurement parameters as carbon dioxide emissions are used as an indicator for the fuel consumption of the internal combustion engine. By the calibration unit 1 with the heating jacket 2 it is possible to provide significant amounts of carbon dioxide with a high precision supported by the scale 7 to measure the gravimetrical amount of carbon dioxide provided to the CVS system through the delivery tube 6 in a short amount of time without blocking the delivery tube 6.

## Description

Subject matter of the present invention is a method and a calibration unit for calibrating a constant volume sampling system for analyzing exhaust emissions and a respective constant volume sampling system. The constant volume sampling system is in particular used for analyzing exhaust of internal combustion engines, e.g. of motor vehicles.

Constant velocity sampling (CVS) is a common technology for gathering samples of internal combustion engines exhausts wherein the exhaust gases produced by an internal combustion engine such as the internal combustion engine of a vehicle are collected as it is driven through a test sequence of accelerations , decelerations, and cruise modes, e.g. on a chassis dynamometer. The collected exhaust is diluted with air to avoid condensation of volatile materials in the exhaust and/or to obtain a comparable volume for several internal combustion engines. Concentrations of pollutants in the total sample are then analyzed for determination of their actual mass.

To obtain reliable results it is necessary to calibrate the process both regarding their flow characteristics (e.g. regarding the volumes, mass flows, etc.) and regarding the accuracy of the analysis. To obtain the latter it is known to use propane as a calibration gas. Here, a known mass of propane is added to the gas flow in the process and the gravimetrically known mass of propane is subtracted from the CVS measured mass and then divided by the gravimetric mass to determine the percent accuracy of the system. This is used as a correction factor to correct the CVS measured data.

Using propane as a calibration gas has the disadvantage that the requirements laid down in different jurisdictions differ so that results gathered e.g. for complying to regulations in the European Union cannot be used immediately to comply for regulations e.g. in the United States of America.

Based on this it is an object of the present invention to provide an alternative method for calibrating a constant volume sampling system to overcome the disadvantages known from prior art.

These objects are solved by the features of the independent claims. Dependent claims are directed to preferred embodiments of the present invention.

The method for calibrating a constant volume sampling system for analyzing exhaust emissions according to the present invention, wherein a gas flow is provided to the constant volume sampling (CVS) system, is characterized by a predetermined amount of carbon dioxide being provided to the gas flow in a predetermined time, wherein the carbon dioxide is provided from a carbon dioxide container, wherein the carbon dioxide container is weighed before starting the provision of the carbon dioxide and after ending the carbon dioxide provision to obtain the weight of carbon dioxide provided to the constant volume sampling system, wherein the carbon dioxide container is heated to a predetermined temperature before the predetermined mass flow of carbon dioxide is started.

In the CVS system preferably the exhaust gas is diluted by a further gas, e.g. air, and the (diluted) exhaust gas is collected in specific containers, so called bags. In the past, carbon dioxide was usually not considered to be reliably usable as a calibration gas for the CVS process as it was not possible to provide a significant amount of carbon dioxide in a defined manner. With the present invention it is possible to provide a significant amount of carbon dioxide, e.g. 100 to 5,000 grams, preferably 250 to 2,500 grams or even more, in a short time period, e.g. in the range of 6 minutes allowing a reliable calibration of the CVS process. The amount of carbon dioxide to be provided depends on the internal combustion engine to be tested and the consumption of fuel of the engine. The internal combustion engine might be used to power motor vehicles or standalone engines, e.g. for electrical generators.

The internal combustion engines can be used to power cars, lorries, off road vehicles, e.g. forestry machinery, or boats or ships. The time period used for providing the carbon dioxide is preferably in the range of 10 minutes and less. By use of a high definition scale it is possible to determine the mass of carbon dioxide introduced into the gas flow accurately, e.g. with a precision of 0.01 grams and less. The carbon dioxide container is preferably a pressurized carbon dioxide container like e.g. a carbon dioxide bottle, in which the carbon dioxide is preferably stored at pressures of up to 70 bar. Higher pressures are possible.

The predetermined temperature is preferably in the range of 30°C and less, in particular in the range of 20°C and 30°C, preferably between 25°C to 30°C. The time during which carbon dioxide is provided varies depending on the amount of carbon dioxide to be provided to the system, in particular in the range of 60 seconds to 500 seconds, or even up to 1,000 seconds for high amounts of carbon dioxide of more than 2,500 grams.

By heating up the carbon dioxide in the carbon dioxide container a situation can be avoided in which the carbon dioxide undergoes a phase change to in part solid carbon dioxide due to the temperature and pressure drop while expanding the carbon dioxide from the pressurized state. This prevents the calibration unit from clogging or blocking which would reduce or stop the delivery of carbon dioxide with the effect that not the full necessary amount of carbon dioxide is delivered to the CVS system. The problem of clogging or blocking the system has prevented the use of carbon dioxide gas as calibration gas for CVS systems in the past.

By using a high precision mass flow controller like a so called Bronkhorst controller the amount and the mass flow of the carbon dioxide delivered to the gas flow can be defined even more precisely.

In the CVS process the known mass or amount of carbon dioxide is subtracted from the CVS measured mass and then divided by the mass known from the calibration process to determine the percent accuracy of the system. This is used as a correction factor to correct the CVS measured data. By applying carbon dioxide as the calibration gas in particular the precision of the carbon dioxide measurement is improved significantly. The calibration method can be used to provide carbon dioxide to a gas flow generated by an actual internal combustion engine. Likewise, it can be used to calibrate the CVS method independently of an internal combustion engine. In this case the gas flow is not comprising exhaust generated by an internal combustion engine but is a gas flow of a predetermined composition like e.g. air or nitrogen. As the composition of the exhaust of internal combustion engines is depending i.a. on environmental variables such as the temperature, moisture etc. a calibration independent of a specific internal combustion engine is advantageous.

To determine the temperature up to which the carbon dioxide container is being heated several approaches can be used individually or at least in part commonly. According to one improvement the temperature is determined based on the amount of carbon dioxide and the time during which the carbon dioxide is to be provided. This allows to consider the temperature drop based on the extraction of the amount of carbon dioxide in the respective time when determining the temperature. According to a second approach the temperature is determined based on the difference between the pressure in the carbon dioxide container before starting the provision of the carbon dioxide and the pressure at which the carbon dioxide is provided to the gas flow. This allows to consider the influence of the pressure difference on the temperature drop when expanding the carbon dioxide from the carbon dioxide container to the pressure at which it is provided to the gas flow. According to a third approach the temperature is determined such that during the provision of the carbon dioxide to the constant volume sampling system the temperature and pressure while extracting the carbon dioxide from the carbon dioxide container is such that the carbon dioxide is provided in gaseous form during the whole provision process. By avoiding the triple point in the pressure vs. temperature diagram of carbon dioxide it is possible to ensure that no solid carbon dioxide is generated while expanding the pressurized carbon dioxide. This ensures that the system is not clogged or blocked ensuring that the total amount of carbon dioxide necessary for the calibration is completely delivered to the gas flow.

According to a further improvement the carbon dioxide container is heated by induction heating.

Induction heating allows to transfer a high heating power on short time scales. It is easily controllable. This allows to easily and efficiently control the temperature of the carbon dioxide container.

According to a further improvement the carbon dioxide container is heated electrically.

An electric heating that can be e.g. a heating jacket is easily and precisely controllable by controlling the electric power provided to the system allowing a reliable control of the temperature of the carbon dioxide container with necessary temperature changes being performed on a short timescale.

According to a further improvement the heating of the carbon dioxide container is continued during at least a part of time during which carbon dioxide is provided.

This allows to introduce a part of the energy that is released during expansion of the carbon dioxide during the expansion of the carbon dioxide to further reduce the risk of blocking and clogging. This is in particular advantageous, if large amounts of carbon dioxide have to be provided. The heating is preferably continued throughout the whole time period during which carbon dioxide is provided.

The heating is preferably continued during the provision of the carbon dioxide if an amount of 1,800 grams and more, in particular 2,000 grams and more, is to be provided.

According to a further aspect a calibration unit for calibrating a constant volume sampling system is proposed, comprising
- a heating jacket surrounding a space allowing to accommodate a carbon dioxide container;
- a scale on which the heating jacket is disposed;
- a delivery tube for connecting the carbon dioxide container with the constant volume sampling system; and
- a temperature sensor for measuring the temperature in the heating jacket.

By the heating jacket, preferably an electrically heatable heating jacket, a carbon dioxide container like a pressurized carbon dioxide container situated inside the space can be heated to the predetermined temperature. By the scale the carbon dioxide container can be weighed before and after the provision of the carbon dioxide to the CVS system to gravimetrically determine the mass of carbon dioxide provided to the CVS system. The carbon dioxide is in use provided to the gas flow of a CVS system through the delivery tube. The temperature sensor allows to determine the temperature in the heating jacket. It can be disposed on the carbon dioxide container itself to allow the measurement of the temperature of the carbon dioxide container itself.

According to a preferred embodiment the calibration unit comprises a control unit for controlling the heating jacket, said control unit being connected at least to the heating jacket and the temperature sensor. The control unit determines the heating power provided to the heating jacket based at least on the data of the temperature sensor.

Preferably, the control unit is designed and adapted for implementing the calibration method according to the present invention.

Preferably, the heating jacket comprises an inductional heater or an electrical heater.

Furthermore, a constant volume sampling system, comprising at least one calibration unit according to the present invention is proposed.

The details and advantages disclosed in connection with the method according to the present invention can be transferred and applied to the calibration unit and the constant volume sampling system of the present invention and vice versa, respectively.

It should be noted that the individual features specified in the claims may be combined with one another in any desired technologically reasonable manner and form further embodiments of the invention. The specification, in particular taken together with the figure, explains the invention further and specifies particularly preferred embodiments of the invention. Particularly preferred variants of the invention and the technical field will now be explained in more detail with reference to the enclosed figures. It should be noted that the exemplary embodiment shown in the figures is not intended to restrict the invention. The figures are schematic and may not be to scale. The figures display:
- Fig. 1: a layout of an example of a calibration unit for calibrating a constant volume sampling system; and
- Fig. 2: a constant volume sampling system with a calibration unit.

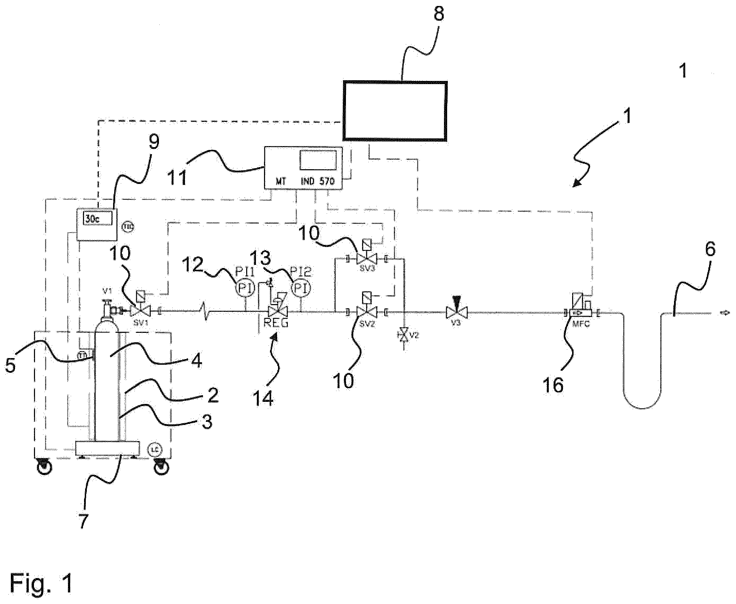

Fig. 1 shows the layout of an example for a calibration unit 1 for calibrating a constant volume sampling system. With reference to this figure the method for calibrating a constant volume sampling system is explained.

The calibration unit 1 comprises a heating jacket 2 surrounding a space 3 allowing to accommodate a carbon dioxide container 4. The carbon dioxide container 4 is a pressurized gas container or gas bottle in which the carbon dioxide is stored at high pressures of up to 70 bar. The heating jacket 2 allows to generate heat electrically. By controlling the electrical power consumed in the heating jacket 2 the temperature of the carbon dioxide container 4 can be controlled. A temperature sensor 5 is disposed on the carbon dioxide container 4 to measure the temperature in the heating jacket 2, and, in particular the temperature of the carbon dioxide container 4.

Due to the expansion of the carbon dioxide when being provided from the carbon dioxide container 4 the carbon dioxide is cooled as is the carbon dioxide container 4. By raising the temperature of the carbon dioxide container 4 and of the carbon dioxide within the carbon dioxide container 4 the temperature of the carbon dioxide provided during the calibration process can be raised. Therefore, situations can be avoided in which the carbon dioxide would at least partly turn solid during extraction. In this case the tube used within the calibration unit 1 as well as the delivery tube 6 which is connecting the carbon dioxide container 4 with the constant volume sampling (CVS) system and through which the carbon dioxide is provided to the constant volume sampling system while calibrating the same could be blocked. This is to be avoided as this would disturb the calibration process. By the delivery tube 6 the carbon dioxide is provide to the gas flow in the CVS system which is exhaust gas before or after dilution with air.

Furthermore, the calibration unit 1 comprises a scale 7 on which the heating jacket 2, and, therefore, the carbon dioxide container 4, is disposed. With the scale 7 the weight of the carbon dioxide container 4 can be measured before and after the provision of carbon dioxide to the CVS system which allows to measure gravimetrically the amount of carbon dioxide provided to the CVS system.

When calibrating the CVS system, the temperature of the heating jacket 2 is raised before the provision of carbon dioxide from the carbon dioxide container 4 is started. The temperature of the heating jacket 2 is regulated to a temperature that ensures that the carbon dioxide container 4 reaches a predetermined temperature. This temperature is determined based on the difference between the pressure in the carbon dioxide container 4 before starting the provision of the carbon dioxide and the pressure at which the carbon dioxide is provided to the gas flow through the delivery tube 6. Furthermore, the amount of carbon dioxide which is to be provided or delivered to the CVS process is taken into consideration when determining the temperature. The temperature is determined such that during the provision of the carbon dioxide to the constant volume sampling system the temperature and pressure while extracting the carbon dioxide from the carbon dioxide container is such that the carbon dioxide is provided completely in gaseous form during the whole calibration process. This means the temperature of the carbon dioxide container 4 is set such that in the pressure vs. temperature diagram the region of solid carbon dioxide is not reached.

The calibration unit 1 further comprises a control unit 8 for controlling the heating jacket 2, said control unit 8 being connected at least to the heating jacket 2 and the temperature sensor 5 via a temperature controller 9. The control unit 8 determines the temperature up to which the carbon dioxide container 4 is to be heated based on the data mentioned above and sets the heating power of the heating jacket 2 accordingly via the temperature controller 9. After the predetermined temperature has been reached the control unit 8 initiates the provision of the carbon dioxide by controlling several valves 10 via a sub-control unit 11. This allows the provision of a significant amount of carbon dioxide (e.g. 250 to 2,500 grams) in a relative short amount of time (e.g. 6 minutes and less) without blocking the delivery tube 6. The control unit 8 is connected to the scale 7 via the sub-control unit 11. Control unit 8, valve controller 9 and sub-control unit 11 are displayed here as separate units, usually they are provided as a single control unit 8 performing all the necessary steps of the respective calibration method.

The calibration unit 1 further comprises a first pressure sensor 12 and a second pressure sensor 13 which are disposed upstream of a pressure regulator 14 in the case of the first pressure sensor 12 measuring the pressure delivered from the carbon dioxide container 4 and downstream of said pressure regulator 14 in the case of the second pressure sensor 13 measuring the pressure at which the carbon dioxide is provided towards the delivery tube 6. The pressure regulator 14 is connected to the control unit 8 as well. By control of the valves the control unit 8 can control the mass flow of carbon dioxide through the delivery tube 6. Furthermore, the calibration unit 1 comprises a mass flow controller 16 allowing a precise control of the mass flow of carbon dioxide into the delivery tube 6. The mass flow controller 16 is connected to and controlled by the control unit 8 as well.

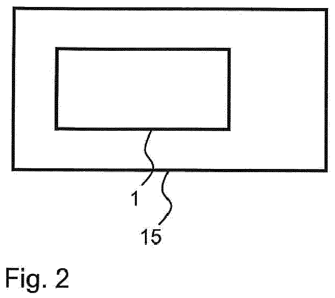

Fig. 2 displays very schematically a constant volume sampling system 15 with a calibration unit 1 according to the present invention.

The calibration unit 1 for calibrating a constant volume sampling (CVS) system comprises a heating jacket 2 by which the carbon dioxide container 4 and the carbon dioxide therein can be heated before the provision to the CVS system starts, thus avoiding blocking the system due to solid carbon dioxide being generated due to the temperature drop originating in the expansion of the carbon dioxide during extraction from the compressed carbon dioxide container 4.

The use of carbon dioxide as a calibration gas for the CVS process has significant advantages compared to the use of propane for the calibration process. On the one hand the legal requirements for carbon dioxide are internationally more harmonized than for propane, in particular in the European Union and the United States of America they are identical. On the other hand, the use of carbon dioxide increases the measurement accuracy in particular for carbon dioxide which is nowadays one of the most important measurement parameters as carbon dioxide emissions are used as an indicator for the fuel consumption of the internal combustion engine. By the calibration unit 1 with the heating jacket 2 it is possible to provide significant amounts of carbon dioxide with a high precision supported by the scale 7 to measure the gravimetrical amount of carbon dioxide provided to the CVS system through the delivery tube 6 in a short amount of time without blocking the delivery tube 6.

### Reference signs

- 1: calibration unit
- 2: heating jacket
- 3: space
- 4: carbon dioxide container
- 5: temperature sensor
- 6: delivery tube
- 7: scale
- 8: control unit
- 9: temperature controller
- 10: valve
- 11: sub control unit
- 12: first pressure sensor
- 13: second pressure sensor
- 14: pressure regulator
- 15: constant volume sampling system
- 16: mass flow controller

## Claims

1. Method for calibrating a constant volume sampling system (15) for analyzing exhaust emissions, wherein a gas flow is provided to the constant volume sampling system (15), **characterized in that** a predetermined amount of carbon dioxide is provided to the gas flow in a predetermined time, wherein the carbon dioxide is provided from a carbon dioxide container (4), wherein the carbon dioxide container (4) is weighed before starting the provision of the carbon dioxide and after ending the carbon dioxide provision to obtain the weight of carbon dioxide provided to the constant volume sampling system (15), wherein the carbon dioxide container (4) is heated to a predetermined temperature before the predetermined mass flow of carbon dioxide is started.

2. Method according to claim 1, wherein the temperature is determined based on the amount of carbon dioxide and the time during which the carbon dioxide is to be provided.

3. Method according to one of the preceding claims, wherein the temperature is determined based on the difference between the pressure in the carbon dioxide container before starting the provision of the carbon dioxide and the pressure at which the carbon dioxide is provided to the gas flow.

4. Method according to one of the preceding claims, wherein the temperature is determined such that during the provision of the carbon dioxide to the constant volume sampling system the temperature and pressure while extracting the carbon dioxide from the carbon dioxide container is such that the carbon dioxide is provided in gaseous form during the whole provision process.

5. Method according to one of the preceding claims, wherein the carbon dioxide container is heated by induction heating.

6. Method according to one of claims 1 to 4, wherein the carbon dioxide container is heated electrically.

7. Method according to claim 5 or 6, wherein the carbon dioxide container is heated using a heating jacket (2).

8. Method according to one of the preceding claims, wherein the heating of the carbon dioxide container (4) is continued during at least a part of time during which carbon dioxide is provided.

9. Calibration unit (1) for calibrating a constant volume sampling system (15), comprising
- a heating jacket (2) surrounding a space (3) allowing to accommodate a carbon dioxide container (4);
- a scale (7) on which the heating jacket (2) is disposed;
- a delivery tube (6) for connecting the carbon dioxide container (4) with the constant volume sampling system (15); and
- a temperature sensor (5) for measuring the temperature in the heating jacket (2).

10. Calibration unit (1) according to claim 9, further comprising a control unit (8) for controlling the heating jacket (2), said control unit (8) being connected at least to the heating jacket (2) and the temperature sensor (5).

11. Calibration unit (1) according to claim 10, wherein the control unit (8) is designed and adapted for implementing the method according to one of the preceding claims.

12. Calibration unit (1) according to one of claims 9 to 11, in which the heating jacket comprises an inductional heater.

13. Calibartion unit (1) according to one of claims 9 to 11, in which the heating jacket comprises an electrical heater.

14. A constant volume sampling system (15), comprising at least one calibration unit (1) according to one of claims 9 to 13.
